# EUROPEAN PATENT SPECIFICATION

(11) **EP 2 108 699 B1**
(45) Date of publication and mention of the grant of the patent: **25.06.2014**
(21) Application number: 08103426.6
(22) Date of filing: 08.04.2008
(51) Int. Cl.: C12N 15/10, C12Q 1/68

(54) **Analytical processing and detection device**
Vorrichtung zur analytischen Verarbeitung und Detektion
Dispositif de traitement et de détection analytique

(43) Date of publication of application: 14.10.2009
(73) Proprietor: F.Hoffmann-La Roche AG, 4070 Basel (CH); Roche Diagnostics GmbH, 68305 Mannheim (DE)
(72) Inventor: Furlan, Alan, 6405 Immensee (CH); Egli, Roman, 8008 Zürich (CH); Roehrig, Sascha, 6006 Luzern (CH)
(74) Representative: Herren, Barbara

(56) References cited:
- WO-A-01/27328
- WO-A1-96/41811
- US-A1- 2006 292 588
- VLIEGER A M ET AL: "QUANTITATION OF POLYMERASE CHAIN REACTION PRODUCTS BY HYBRIDIZATION-BASED ASSAYS WITH FLUORESCENT, COLORIMETRIC, OR CHEMILUMINESCENT DETECTION1" ANALYTICAL BIOCHEMISTRY, ACADEMIC PRESS INC. NEW YORK, vol. 205, no. 1, 15 August 1992 (1992-08-15), pages 1-07, XP000294452 ISSN: 0003-2697

## Description

The present invention relates to an analytical processing and detection device which allows for the detection of a detection signal in the fluid of an analytic receptacle in the presence of magnetic particles.

Commonly, for analytical methods based on gene or protein expression or genetic analysis, analytes in biological samples are prepared by adsorption (binding) to a solid phase, such as magnetic particles, and then further processed to obtain a detection signal, thus obtaining an analytical result that can be used in diagnosis of disease or in the monitoring for treatment.
In the field of PCR diagnostics, the analyte which is bound to the particles is washed and may be eluted prior to further processing. The eluate is then transferred to new receptacles for PCR amplification and detection of amplified PCR product in the liquid. In such a setting, no particles are transferred and PCR amplification and detection are carried out in the absence of the particles, which would disturb the optical detection of the detection signal in the liquid. However, the elution and transfer of the analyte has the disadvantage that the analyte comprised in one reaction receptacle can not be completely transferred due to residual liquid remaining in the reaction receptacle (interstitial volume). In the field of PCR diagnostics, where small amounts of sample are processed and analysed, such incomplete transfer of liquids impairs the sensitivity of the analysis.

WO03/057910 discloses a method of amplifying and optically detecting the detection signal in the liquid phase of receptacles without removal of the magnetic particles. The particles are allowed to settle to the bottom of the receptacles by sedimentation.

### Description

The present invention relates to an analytical processing and detection device comprising
- a thermoblock for receiving at least one reaction receptacle, said rack comprising at least one cavity which can receive said reaction receptacle,
- a magnetic assembly integrated into said thermoblock said magnetic assembly comprising one or more units for exerting a magnetic field on the at least one reaction receptacle comprising magnetic particles capable of binding an analyte contained in a fluid, and wherein said magnetic field generated by at least one magnetic unit causes the magnetic particles to be sequestered to the interior side wall of said at least one reaction receptacle, and
- a detection unit for detecting a signal corresponding to an analyte concentration in the supernatant of said at least one reaction receptacle wherein said detection unit is aligned with said receptacle located in said thermoblock in the presence of the sequestered magnetic particles.

Said device, further described below, can be used in a method for processing the contents of at least one reaction receptacle and determining the amount of an analyte bound to magnetic particles present in each of said reaction receptacles, wherein said analyte can be processed and determined in the presence of the magnetic particles accurately and with high sensitivity by connecting one or more magnetic units to said rack.

The magnetic unit(s) is/are coupled to the rack such that the magnetic particles are sequestered on the side walls of the reaction receptacles. The term "coupled" as used herein relates to any type of connection between magnet and rack. The magnet(s) may be reversibly coupled to said rack. The magnets may, preferably, be stably integrated into the rack.

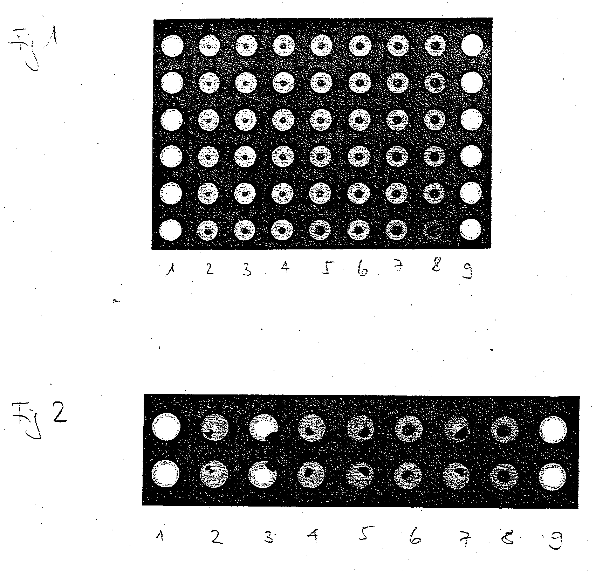

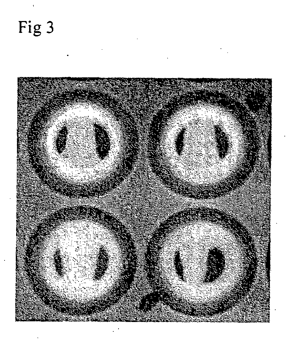

Thus, the analyte can be processed without need for separating the eluate from the magnetic particles, because detection signal is only minimally impaired by the magnetic particles (see Figure 3). The term "separating the eluate from the magnetic particles" is understood to mean that the eluate is separated, and that following separation, is no longer in physical contact with the magnetic particles. Figure 1 shows how the detection signal is impaired by the presence of unsequestered or insufficiently sequestered magnetic particles. Due to magnetic sequestration of the magnetic particles in the receptacle, the loss of material during pipetting of the eluate due to interstitial volume can be avoided. In addition, although the magnetic particles are not removed, the sequestration to the sidewalls of the reaction receptacles reduces a lowering of the signal-to-noise ratio caused by suspended or sedimented particles and, thus, improves the sensitivity of the detection.

The term "reaction receptacle" as used herein relates to a receptacles which can hold a liquid and in which a reaction, such as a chemical or enzymatic reaction can take place. In a preferred embodiment, a microtiter plate comprising a plurality of reaction receptacles is employed, more preferably a 96 well microtiter plate. Shapes of microtiter plates are commonly known. The microtiter plate is preferably white. The white plate improves the efficiency of fluorescence excitation and detection by allowing multiple light reflexion within the beam. The white plates are typically polypropylene plates comprising titanium dioxide as a white color agent. It is important that said plates lack any trace substances which may contribute to plate autofluorescence. The shape of the reaction receptacles, in preferred embodiments, is determined by both thermal and liquid handling considerations.

From the thermal perspective, the shape should be such as to maximize direct thermal contact with the thermal block, in order to increase temperature ramps and thermal equilibration, which would ideally be a high, thin cylinder or a very short, flat cylinder. The walls of the receptacle must have a sufficient inclination angle from vertical such that the microtiter plate can be easily removed from the thermocycler after the PCR amplification. Furthermore, the shape of the receptacle must be wide enough and have a round bottom to avoid bubble formation during liquid dispensing.

The term "magnetic particles" relates to particles comprising a magnetic material which can bind an analyte in a fluid. Said term encompasses magnetic particles with or without a silica surface. Said magnetic particles are preferably magnetic glass particles.

In a preferred embodiment, if the analyte is a nucleic acid, said magnetic glass particles are magnetic glass particles comprising an unmodified silica surface. Preferred embodiments of said magnetic glass particles are disclosed in WO 96/41811. The magnetic glass particles are a solid dispersion of small magnetic cores in glass, i.e. they are glass droplets in which very small magnetic objects are dispersed. Those objects that are referred to as magnetic are drawn to a magnet, i.e. ferri-or ferromagnetic or superparamagnetic materials for instance. Paramagnetic substances are not useful as they are only drawn to a magnet very weakly which is not sufficient for a method according to this invention. Preferred are ferri-or ferromagnetic materials, in particular if they have not yet been premagnetized. Premagnetization in this context is understood to mean bringing in contact with a magnet, which increases the remanence. Preferred magnetic materials are iron or iron oxide as e.g. magnetite (Fe₃O₄) or Fe₂O₃, preferably gamma-Fe₂O₃. In principle, barium ferrite, nickel, cobalt, Al-Ni-Fe-Co alloys or other ferri-or ferromagnetic material could be used. Particularly preferred according to the present invention are the magnetic glass particles described in WO96/41811 or WO00/32762 or WO98/12717.

In a very preferred embodiment of the invention, the magnetic glass particles with an unmodified glass surface have a low iron leach which is essential for the method according to the invention when using magnetic glass particles, as iron is an inhibitor of the subsequent amplification reaction, i.e. iron is an enzymatic inhibitor. Therefore, this is an important feature of the magnetic glass particles with an unmodified glass surface. In the most preferred embodiment of the invention, the magnetic glass particles with an unmodified surface are those described in the European application EP 20 00110165.8 which are also publicly available in the MagNA Pure LC DNA Isolation Kit I (Roche, Mannheim, Germany). The production thereof is summarized below.
The most preferred MGPs according to the invention are manufactured according to the international application EP1154443 which are also provided in the MagNA Pure LC DNA Isolation Kit I (Roche, Mannheim, Germany)). They are also produced by the sol-gel-method as described in the international application (EP1154443) using magnetic objects or pigments with a diameter of about 23 nm (manufactured by CERAC consisting of γ -Fe₂O₃; CERAC: P.O. Box 1178, Milwaukee, Wisconsin 53201-1178 USA; Article-No. I-2012).

According to the present invention, an "analyte" is understood to be any molecule, or aggregate of molecules, including a cell or a cellular component of a virus, found in a sample. Thus, as a non-limiting example, an analyte may be a nucleic acid of interest or a protein of interest which is investigated and its presence or absence, or its concentration in a biological sample is determined as its presence or absence is indicative of a certain condition or disease of a human or animal. Further included in the scope of the term "analyte" are fragments of any such molecule found in a sample. In one preferred embodiment, said analyte is a biological analyte, more preferably a nucleic acid. Said nucleic acid may be RNA or DNA or any derivative thereof. In a more preferred example, said analyte is a virus, more preferably the hepatitis A virus (HAV), hepatitis B virus (HBV), hepatitis C virus (HCV), the human immunodeficiency virus (HIV), the human papilloma virus (HPV), parvovirus BI9, CT/NG. The analyte may also, in a more preferred example, be a bacteria such as MAI or MTB.

The term "fluid" relates to any kind of liquid comprising said magnetic particles capable of binding an analyte. This may be a biological sample fluid as described hereinafter, or a buffered solution. In one embodiment, said buffered solution is an elution buffer. Elution buffers with a low salt content are in particular buffers with a content of less than 0.2 mol/l. In an especially preferred embodiment, the elution buffer contains the substance Tris for buffering purposes, in particular a Tris buffered solution with a pH around 7 or above 7. In another special embodiment, the elution buffer is demineralized water.

The term "rack" as used herein refers to a unit with cavities which can hold at least one reaction receptacle. Said rack is a-thermoblock. This means that the material of said rack preferably has a high thermal dffusivity, which is necessary for fast thermal ramps. This is given at high thermal conductivity preferably of more than 200 W/(mK), more preferably fo at least 221 W/(mK), low heat capacity, preferably less than 1000 J/(kg K), more preferably equal or less than 900 J/(kg K), and low density, preferably less than 12 kg/dm³, more preferably equal or less than 10.5 kg/dm³. In a more preferred embodiment, the material of the rack hereinbefore described comprises aluminum or silver. The thermal conductivity of silver is 429 W/(mK), of aluminum 221 W/(mK). The heat capacity of silver is 232 J/(kg K), of aluminum 900 J/(kg K). The density of silver is 10.5 kg/dm³, of aluminum 2.7 kg/dm³. In a most preferred embodiment, the rack hereinbefore described is a heating block in a thermal cycler. Said heating block is controlled to change between at least two temperatures, preferably between an annealing, an incubation and a denaturation temperature, in one cycle. The temperature of said heating block can be changed rapidly. Cooling may be achieved by contacting said heating block with a heat sink, e.g. a liquid. Or it may be achieved by a thermoelectric element, such as a Peltier element. A combination of an resistive heater and a thermoelectric cooling element, or a thermoelectric element used in both heating and cooling mode are the most frequently used configurations, apart from heating and cooling by using air.

The term "magnetic unit" as used herein relates to any unit which can exert a magnetic field. Said magnetic unit may be an electromagnetic unit or a permanent magnet.

In one embodiment, the magnetic field exerted on the reaction receptacles comprising said magnetic particles as described hereinbefore can be increased or decreased. Such increases of the magnetic field may be achieved by moving a magnet into proximity with the rack holding the reaction receptacles. Consequently, decreases may be achieved by removing said magnet from the rack.

In another embodiment of the invention hereinbefore described, said one or more units for exerting a magnetic field on at least one reaction receptacle comprising magnetic particles in the fluid are one or more permanent magnets which are stably integrated into said rack. In a preferred embodiment, said magnetic units are at least two magnetic units. In a preferred embodiment, said one or more permanent magnets are thermostable at temperatures of up to 140°C. This means that ability of the permanent magnet to generate a magnetic field is not impaired by such high temperatures. This allows the generation of a magnetic field even during thermocycling, which necessitates heating of the contents of the liquid inside the reaction receptacle to temperatures as high as 100°C. The thermal block itself can transiently heat up to temperatures of 110°C. This so-called block overshoot is used to accelerate the equilibration of the liquid to the final temperature. In a more preferred embodiment, said one or more permanent magnets are thermostable at temperatures of up to 180°C. In an even more preferred embodiment, said one or more permanent magnets are thermostable at temperatures of up to 250°C. In a most preferred embodiment, said one or more permanent magnets comprise a SmCo alloy.

The geometry and placement of the permanent magnet hereinbefore described may also affect the efficiency of sequestration of the magnetic particles to the interior side walls of the reaction receptacles, as hereinbefore described. Therefore, in a preferred embodiment, said cavities for receiving reaction receptacles are arranged between at least two permanent magnets such that equal magnetic poles of said permanent magnets face the same cavities.

In one more preferred embodiment of the device hereinbefore described, said two or more permanent magnets are pin-shaped and said magnets extend over the whole width or length of said rack. In another more preferred embodiment, said permanent magnets are pin-shaped, wherein the length of said magnets corresponds approximately to the diameter of a cavity of said rack, and wherein said magnet is arranged between two cavities for receiving reaction receptacles of said rack such that equal magnetic poles of said permanent magnets face the same cavity. In a more preferred embodiment, the magnetic field is adjusted such that the area of the collected beads projected onto a horizontal surface should be minimal. Most preferably, the beads are well focused just below the liquid surface.

The device hereinbefore described is suitable for detecting a signal which is impaired by the presence of magnetic particles. The term "detection unit" therefore relates to a detection unit which can detect a signal which is impaired by the presence of magnetic particles. Preferably, said detection unit is an optical unit. More preferably, said signal is fluorescence. Said detection units may comprise photodiodes or CCD chips (as described, e.g. in WO99/60381 or DFE19748211). Said detection units further comprise a light source for emitting excitation light, such as a 100 watt halogen lamp (WO99/60381) or an array of LEDs. Said detection device my further optionally comprise a beam splitter ((DE10131687, DE10155142). Furthermore, said detection device may comprise Fresnel. lenses (US6246525), field lenses (EP 1681556) or one or more telecentric lenses (US6498690).

Further to the device hereinbefore described, the present invention also relates to an analytical system comprising a device as described hereinbefore.

Such analytical system and device can be used for performing a method for determining the amount of an analyte bound to magnetic particles present in a sample fluid in a reaction receptacle,
said method comprising the the follwing steps:
a) providing a biological sample comprising an analyte in at least one reaction receptacle
b) binding said analyte to magnetic particles in said at least one reaction receptacle;
c) processing said analyte bound to magnetic particles in said at least one reaction receptacle to obtain a detection signal. The detection signal is a signal that is disturbed by the presence of said magnetic particles. In a preferred embodiment, the detection signal is an optical signal. Furthermore, in step c) the at least one reaction receptacle is located in an the device described hereinbefore.
In step d) a supernatant is generated by applying a magnetic field on the contents of said at least one reaction receptacle. The magnetic field causes the magnetic particles to be sequestered. Step d) is followed by step e), in which a detection signal corresponding to an analyte concentration in the supernatant of said reaction receptacle is detected, . preferably by optical detection, whils the magnetic particles form a pellet at the site of the side walls of the reaction receptacle to which the were sequestered. In the method of the present invention, steps c) to e) are carried out in the device described hereinbefore.

Preferably, all steps are automated. Automatized method means that the steps of the automatable method are carried out with an apparatus or machine capable of operating with little or no external control or influence by a human being.

In a preferred embodiment of the invention, the method is in a high-throughput format, i.e. the automated method is carried out in a high-throughput format which means that the methods and the used machine or apparatus are optimized for a high-throughput of > 100 samples in a short time.

The term "supernatant" as used herein relates to the liquid in a reaction receptacle after sequestration of magnetic particles by a magnetic field. Thus, the supernatant refers to the liquid in the reaction receptacles after the magnetic particles were sequestered by the magnetic field, while the magnetic particles form a pellet at the site of the side walls of the reaction receptacles to which they were sequestered.

The term "biological sample" as used herein relates to any sample derived from a biological organism. In an embodiment of the invention, the biological sample comprises viruses or bacterial cells, as well as isolated cells from multicellular organisms as e.g. human and animal cells such as leucocytes, and immunologically active low and high molecular chemical compounds such as haptens, antigens, antibodies and nucleic acids, blood plasma, cerebral fluid, sputum, stool, biopsy specimens, bone marrow, oral rinses, blood serum, tissues, urine or mixtures thereof. Thus, the biological sample may be either solid or fluid. In a preferred embodiment of the invention the biological sample is a fluid from the human or animal body. A biological sample which is a fluid is also called a sample fluid. Preferably the biological sample is blood, blood plasma, blood serum or urine. The blood plasma is preferably EDTA-, heparin-or citrate-treated blood plasma. In an embodiment of the invention the biological sample comprises bacterial cells, eukaryotic cells, viruses or mixtures thereof. In a preferred embodiment of the invention, the virus is the hepatitis A virus (HAV), hepatitis B virus (HBV), hepatitis C virus (HCV), the human immunodeficiency virus (HIV), the human papilloma virus (HPV), parvovirus B19, CT/NG, CMV, and the bacteria are MTB or MAI. The biological sample can also be of a type used for environmental analysis, food analysis or molecular biology research, e.g. from bacterial cultures or phage lysates.

The biological sample comprising a mixture of biological compounds comprising non-target and a target nucleic acid needs not to be lysed, when the biological sample can be used without pretreatment in the method according to the invention. However, preferably a biological sample comprising non-target nucleic acids and a target nucleic acid is lysed to create a mixture of biological compounds comprising non-target and a target nucleic acid. Therefore, the biological compounds, non-target nucleic acids and the target nucleic acid contained in the biological sample are released thereby creating a mixture of biological compounds comprising non-target nucleic acids and the target nucleic acid. Procedures for lysing biological samples are known by the expert and can be chemical, enzymatic or physical in nature. A combination of these procedures is applicable as well. For instance, lysis can be performed using ultrasound, high pressure, shear forces, alkali, detergents or chaotropic saline solutions, or proteases or lipases. For the lysis procedure to obtain nucleic acids, special reference is made to Sambrook et al.: Molecular Cloning, A Laboratory Manual, 2nd Addition, Cold Spring Harbour Laboratory Press, Cold Spring Harbour, NY and Ausubel et al.: Current Protocols in Molecular Biology 1987, J. Wiley and Sons, NY.

The method of the present invention comprises the step of binding an analyte to magnetic particles in a reaction receptacle

The magnetic glass particles used in the present invention may be provided in different formulations essentially as described in European patent publication EP1154443. It is possible to provide them in the form of a tablet, as a powder or preferably as a suspension. In a preferred embodiment of the invention these suspensions contain between 5 to 60 mg/ ml magnetic glass particles (MGPs). In another embodiment of the invention the silica-containing material is suspended in aqueous buffered solutions which may optionally contain a chaotropic agent in a concentration of between 1 and 8 mol/l, preferably 2 and 8 mol/l, and preferably between 2 and 6 mol/l, more preferably between 4 and 6 mol/l. Chaotropic salts are sodium iodide, sodium perchlorate, guanidinium thiocyanate, guanidinium isothiocyanate or guanidinium hydrochloride. A chaotropic agent according to the present invention is any chemical substance which will disturb the ordered structure of liquid water and will have the effect that DNA or RNA will bind to the MGPs according to the present invention if this agent is present in the DNA or RNA containing solution. Other compounds known to the expert in the field are also possible. The purification effect results from the behavior of DNA or RNA to bind to material with a glass surface under these conditions i.e. in the presence of certain concentrations of a chaotropic agent, higher concentrations of organic solvents or under acidic conditions. To bring the mixture of biological compounds comprising non-target nucleic acids and the target nucleic acid, the glass beads with an unmodified glass surface are added to the mixture and incubated for a period of time sufficient for the binding to occur. Experts are usually familiar with the duration of the incubation step. This step can be optimized by determining the quantity of immobilized nucleic acids on the surface at different points in time. Incubation times of between 10 seconds and 30 minutes can be appropriate for nucleic acids.

One procedure for binding a target nucleic acid (and also the non-target nucleic acids) to magnetic glass particles can be described in detail as follows. Other procedures are also known to the skilled person and may be used as well. While the term "analyte" as defined hereinbefore relates to the target nucleic acid, both target and non-target nucleic acids may be bound to the magnetic glass particles. In one embodiment, the target nucleic acids are bound specifically to the magnetic particles. This specific binding may be mediated, as a non-limiting example, by an oligonucleotide with a sequence which is complementary to the target sequence, whereby said oligonucleotide is firmly bound to the magnetic glass particles. The binding of target and/or non-target nucleic acid is preferably performed in the presence of chaotropic salts in concentrations as described hereinbefore.

Following binding of the analyte to the magnetic particles, the magnetic particles with the bound analyte are separated from the sample. Optionally, the magnetic particles with the bound analyte may be washed with a washing buffer.

This may be by separating the material bound to the magnetic particles by applying a magnetic field. For instance, the magnetic particles can be pulled to the wall of the vessel in which incubation was performed. The liquid containing the biological compounds that were not bound to the magnetic particles can then be removed. Therefore, the method according to the invention contains the step of separating said material with said bound non-target nucleic acids and/or said bound target nucleic acid from the non-bound biological compounds.

The removal procedure used depends on the type of vessel in which incubation was performed. Suitable steps include removing the liquid via pipetting or aspiration. The material with the bound DNA or RNA may then be washed at least once, preferably with a mixture of 70 volume parts ethanol with 30 volume parts water ("70 % Ethanol") or in an acidic wash solution as described in WO 99/40098. A wash solution is used that does not cause the nucleic acids and the target nucleic acid to be released from the material surface but that washes away the undesired contaminants as thoroughly as possible. This wash step preferably takes place by incubating the magnetic glass particles with the unmodified silica surface with the bound nucleic acids and the target nucleic acid. The material is preferably resuspended during this step. The contaminated wash solution is preferably removed just as in the binding step described above. After the last wash step, the material can be dried briefly in a vacuum, or the fluid can be allowed to evaporate. A pretreatment step using acetone may also be performed.

The solution containing the purified analyte is now ready to be further processed. The term "processing" as used herein relates to any manipulation of the analyte, e.g. by chemical or biochemical manipulation, which leads to the production of a detection signal. In a preferred embodiment of the method hereinbefore described, the processing of said nucleic acid comprises amplification. Amplification is a well known method to multiply copies of a specific sequence of RNA or DNA which can then be detected and analysed quantitatively or qualitatively. In a preferred embodiment, said processing comprises elution of said analyte from the magnetic beads into the fluid in said reaction receptacle. For elution to take place, the material with the unmodified silica surface is resuspended in a solution with no or only a low amount of chaotropic agent and/ or organic solvent. Alternatively, the suspension can be diluted with a solution with no or only a low amount of chaotropic agent and/ or organic solvent. Buffers of this nature are known from DE 3724442 and Analytical Biochemistry 175 (1988) 196-201. The elution buffers with a low salt content are in particular buffers with a content of less than 0.2 mol/l. In an especially preferred embodiment, the elution buffer contains the substance Tris for buffering purposes, in particular a Tris buffered solution with a pH around 7 or above 7. In another special embodiment, the elusion buffer is demineralized water.

The term "processing unit" relates to a unit in which the analyte can be processed for detection. Such a processing unit may be a thermal cycler. In a preferred embodiment, said processing unit is the analytical processing and detection device hereinbefore described. Thus, at least steps c) to e) of the method of the present invention can be carried out in said processing and detection device.

For amplification, all reagents necessary for amplification are added to the solution comprising the analyte. Otherwise, a solution containing all reagents necessary for amplification is added to the suspension of the material with the unmodified silica surface and the target nucleic acid.

In a preferred embodiment of the invention, the target nucleic acid is amplified with the polymerase chain reaction (PCR). The amplification method may also be the Ligase Chain Reaction (LCR, Wu and Wallace, Genomics 4 (1989)560-569 and Barany, Proc. Natl. Acad. Sci. USA 88 (1991)189-193); Polymerase Ligase Chain Reaction (Barany, PCR Methods and Applic. 1 (1991)5-16); 20 Gap-LCR (PCT Patent Publication No. WO 90/01069); Repair Chain Reaction (European Patent Publication No. EP 439,182 A2), 3SR (Kwoh, et al., Proc. Natl. Acad. I Sci. USA 86 (1989)1173-1177; Guatelli, et al., Proc. Natl. Acad. Sci. USA 87 (1990)1874-1878; PCT Patent Publication No. WO 92/0880A), and NASBA (U.S. Pat. No. US 5,130,238). Further, there are strand displacement amplification (SDA), transciption mediated amplification (TMA), and Q0-amplification (for a review see e.g. Whelen and Persing, Annul Rev. Microbiol. 50 (1996) 349-373; Abramson and Myers, Current Opinion in Biotechnology 4 (1993)41-47).

A preferred detection signal of the method hereinbefore described is an optical detection signal. More preferably, said optical detection signal is fluorescence.

The target nucleic acid may be detected by measuring the intensity of fluorescence light during amplification. This method entails the monitoring of real time fluorescence. A particularly preferred method exploiting simultaneous amplification and detection by measuring the intensity of fluorescent light is the TaqMan method disclosed in WO92/02638 and the corresponding US patents US 5,210,015, US 5,804,375, US 5,487 972. This method exploits the exonuclease activity of a polymerase to generate a signal. In detail, the target nucleic acid is detected by a process comprising contacting the sample with an oligonucleotide containing a sequence complementary to a region of the target nucleic acid and a labeled oligonucleotide containing a sequence complementary to a second region of the same target nucleic acid strand, but not including the nucleic acid sequence defined by the first oligonucleotide, to create a mixture of duplexes during hybridization conditions, wherein the duplexes comprise the target nucleic acid annealed to the first oligonucleotide and to the labeled oligonucleotide such that the 3'-end of the first oligonucleotide is adjacent to the 5'-end of the labeled oligonucleotide. Then this mixture is treated with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments. The signal generated by the hydrolysis of the labeled oligonucleotide is detected and/ or measured. TaqMan technology eliminates the need for a solid phase bound reaction complex to be formed and made detectable. In more general terms, the amplification and/ or detection reaction of the method according to the invention is a homogeneous solution-phase assay. A further preferred method is in the Lightcycler^{™} format (see e.g. US 6,174,670).

The detection methods may include but are not limited to the binding or intercalating of specific dyes as ethidiumbromide which intercalates into the doublestranded DNA and changes its fluorescence thereafter. An excitation beam from a light source is directed onto the liquid in said reaction receptacle. The emission of fluorescent light from the liquid following excitation is indicative of a positive signal, and can be detected and quantitated.

In an embodiment different from the embodiment in which said analyte is a nucleic acid, said analyte is a polypeptide. In a preferred embodiment, said processing of said polypeptide in step c) comprises the steps of
c1) contacting said analyte bound to magnetic particles with a detection molecule, and
c2) eluting said detection molecule bound to said analyte to obtain a detection signal in a fluid in said reaction receptacles. In a more preferred embodiment, said detection molecule is a labeled antibody which can bind said analyte.

For any of the embodiments of the method hereinbefore described, said magnetic field is produced by a magnetic assembly integrated into a thermoblock. Said magnetic assembly integrated into a thermoblock can be any of the embodiments or preferred, more preferred or most preferred embodiments hereinbefore described.

Figures
Fig. 1 shows an image of emitted fluorescent light from wells of a 96 well plate with different amounts of magnetic particles.
Fig. 2 shows the effects of different degrees of sequestration of the MGPs.
Fig. 3 shows schematic views of a preferred arrangement of the magnets in the heat block
Fig. 4 shows one possible arrangement of magnets and cavities.
Fig. 5 shows a device comprising a detection unit, a rack holding a receptacle and magnets coupled to said rack.

### Examples

Figure 1 shows an image of the fluorescent light emitted from the wells of a 96 well microtiter plate (not all wells are shown). Each well comprises an identical volume and concentration of fluorochrome (50 µl of 50 nM fluorescein and 50 µl of Tris buffer) and, from left to right, an increasing amount of magnetic particles (Roche Magnetic Bead particles as used for Cobas Taqman^{™} and Magnapure^{™} assays). The first and last columns of wells comprise fluorescent dye without particles. It can be seen that the fluorescence intensity is highest in the absence of magnetic particles and decreases with increasing amount of sedimented magnetic particles. From left to right, each well contains 0, 1, 2, 4, 6, 8, 12, 16 and 0 mg of magnetic particles, respectively.

Figure 2 shows the effects of different degrees of sequestration of the magnetic particles. Fluorescence intensity in column 3 is significantly increased. Furthermore, Figure 3 shows that increased intensity can be achieved in multiple wells.

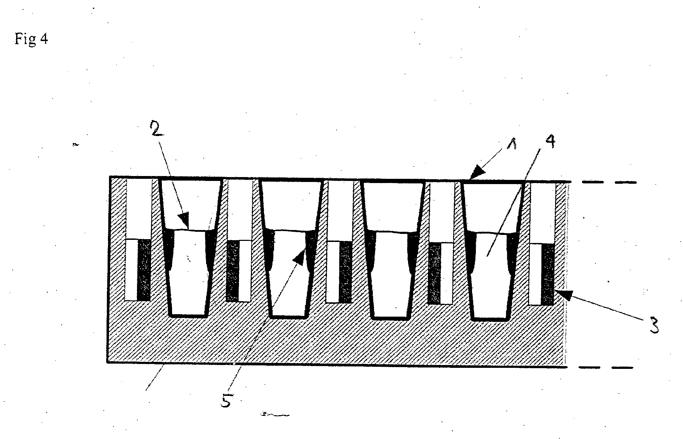

Figure 4 shows a schematic view of a reaction receptacle (1) comprising a liquid (4). A magnetic unit (3) is located below the meniscus of the liquid (2) such that the magnetic particles (5) are sequestered to the side wall of the reaction receptacle. In order to minimize the loss of emitted light collected by a detector which is located above the well, the area of the collected beads projected onto a horizontal surface should be minimal. This can be achieved by adjusting the magnetic field such that the beads are well focused just below the liquid surface. If the beads are pulled above the liquid surface, they tend to generate a bulge which blocks part of the excitation and emission light.

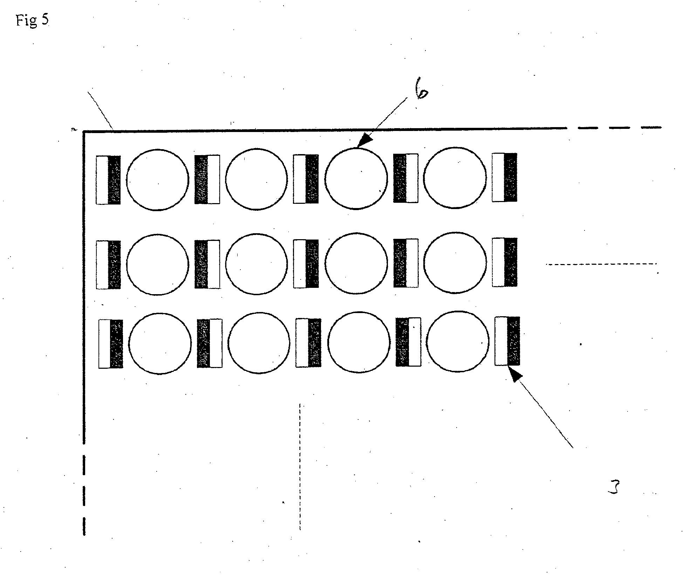

Fig. 5 shows a schematic view of one possible arrangement of cavities (6) and magnetic units (3), whereby the cavities are located between equal magnetic poles (black, white parts) of the magnetic units.

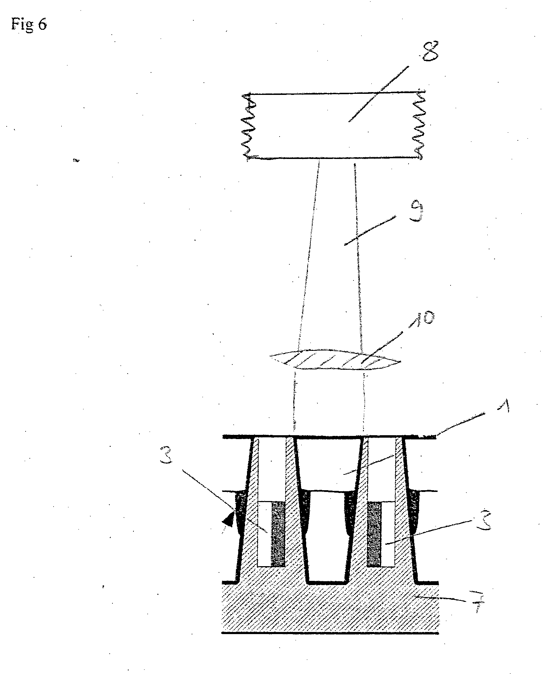

Fig. 6 shows a device with a receptacle (1) which may be an individual receptacle or part of a multiwell plate. Said receptacle is held in a rack (7), and located between equal poles of magnetic units (3). The device also comprises a detection unit (8) which detects the emission light (9). Lenses (10) may be located between the receptacle and the detection device.

## Claims

1. An analytical processing and detection device comprising
- a Thermoblock for receiving at least one reaction receptacle, said Thermoblock comprising at least one cavity which can receive said reaction receptacle,
- a magnetic assembly integrated into said Thermoblock, said magnetic assembly comprising one or more units for exerting a magnetic field on the at least one reaction receptacle comprising magnetic particles capable of binding an analyte contained in a fluid, and wherein said magnetic field generated by at least one magnetic units causes the magnetic particles to be sequestered to the interior side wall of said at least one reaction receptacle, and
- a detection unit for detecting a signal corresponding to an analyte concentration in the supernatant of said at least one reaction receptacle, wherein said detection unit is aligned with said receptacle located in said thermoblock in the presence of the sequestered magnetic particles.

2. The device according to claim 1, wherein the material of said Thermoblock comprises aluminum or silver.

3. The device according to claims 1 to 2, wherein said magnetic field can be increased or decreased.

4. The device according to any one of claims 1 to 3, wherein said one or more units for exerting a magnetic field on at least one reaction receptacle comprising magnetic particles in the fluid are one or more permanent magnets which are integrated into said Thermoblock.

5. The device according to claim 4, wherein said one or more permanent magnets are thermostable at temperatures of up to 140°C, preferably of up to 184°C, more preferably of up to 250°C.

6. The device according to claim 4, wherein said one or more permanent magnets comprise a SmCo alloy.

7. The device according to any one of claims 4 to 6, wherein said cavities for receiving reaction receptacle of said Thermoblock are arranged between at least two of said permanent magnets such that equal magnetic poles of said permanent magnets face the same cavities.

8. The device according to any one of claim 7, wherein said permanent magnets are located below the meniscus of a liquid comprised in a reaction receptacle which is held by said Thermoblock.

9. The device according to any one of claims 1 to 8, wherein said detection unit is an optical detection unit.

10. A method for determining the amount of an analyte bound to magnetic particles present in a sample fluid in a reaction receptacle,
said method comprising the steps of
a) providing a biological sample comprising an analyte in at least one reaction receptacle
b) binding said analyte to magnetic particles in said at least one reaction receptacle;
c) processing said analyte bound to magnetic particles in said at least one reaction receptacle to obtain a detection signal, said detection signal being a signal that is disturbed by the presence of said magnetic particles, wherein said at least one reaction receptacle is located in a device according to claims 1to 9;
d) generating a supernatant by applying a magnetic field on the contents of said at least one reaction receptacle and sequestering said magnetic particles;
e) detecting said detection signal corresponding to an analyte concentration in the supernatant of said reaction receptacle while the magnetic particles form a pellet at the site of the side walls of the reaction receptacle to which they were sequestered,
wherein steps c) to e) are carried out in the same analytical processing unit and the reaction receptacle is located in the thermoblock of the device according to claims 1 to 9.

11. The method of claim 10, wherein said analyte is a nucleic acid or a polypeptide.

12. The method of claims 10 and/or 11, wherein processing of said nucleic acid comprises amplification.

13. The method according to any one of claims 10 to 12, wherein said detection signal is an optical detection signal, preferably fluorescence.

14. The method according to any one of claims 10 to 13, wherein said processing comprises elution of said analyte from the magnetic beads into the fluid in said reaction receptacle.

15. The method according to claim 11, wherein said processing of said polypeptide in step c) comprises the steps of
c1) contacting said analyte bound to magnetic particles with a detection molecule, preferably a labeled antibody which can bind said analyte, and
c2) eluting said detection molecule bound to said analyte to obtain a detection signal in a. fluid in said reaction receptacles.

## Patentansprüche

1. Analytische Bearbeitungs- und Nachweiseinheit, umfassend
- einen Thermoblock zum Aufnehmen wenigstens eines Reaktionsgefäßes, wobei der Thermoblock wenigstens einen Hohlraum umfasst, der das Reaktionsgefäß aufnehmen kann,
- eine magnetische Baugruppe, die in den Thermoblock integriert ist, wobei die magnetische Baugruppe eine oder mehrere Einheiten zum Anwenden eines Magnetfelds auf das wenigstens eine Reaktionsgefäß, das zum Binden eines in einem Fluid enthaltenden Analyten fähige magnetische Partikel enthält, umfasst und wobei das von wenigstens einer magnetischen Einheit erzeugte Magnetfeld bewirkt, dass die magnetischen Partikel an die innere Seitenwand des wenigstens einen Reaktionsgefäßes abgesondert werden, und
- eine Nachweiseinheit zum Nachweisen eines Signals, das einer Analytenkonzentration in dem Überstand des wenigstens einen Reaktionsgefäßes entspricht, wobei die Nachweiseinheit bezogen auf das Gefäß ausgerichtet ist, das in Gegenwart der abgesonderten magnetischen Partikel in dem Thermoblock angeordnet ist.

2. Einheit gemäß Anspruch 1, wobei das Material des Thermoblocks Aluminium oder Silber umfasst.

3. Einheit gemäß Ansprüchen 1 bis 2, wobei das Magnetfeld erhöht oder verringert werden kann.

4. Einheit gemäß einem der Ansprüche 1 bis 3, wobei die eine oder mehreren Einheiten zum Anwenden eines Magnetfelds auf wenigstens ein Reaktionsgefäß, das magnetische Partikel in dem Fluid enthält, ein oder mehrere Permanentmagnete sind, die in den Thermoblock integriert sind.

5. Einheit gemäß Anspruch 4, wobei der eine oder die mehreren Permanentmagnete bei Temperaturen von bis zu 140 °C, vorzugsweise von bis zu 180 °C, bevorzugter von bis zu 250 °C, wärmestabil sind.

6. Einheit gemäß Anspruch 4, wobei der eine oder die mehreren Permanentmagnete eine SmCo-Legierung umfassen.

7. Einheit gemäß einem der Ansprüche 4 bis 6, wobei die Hohlräume des Thermoblocks zum Aufnehmen von Reaktionsgefäßen der Thermoblöcke zwischen wenigstens zwei der Permanentmagnete so angeordnet sind, dass gleiche Magnetpole der Permanentmagnete auf die gleichen Hohlräume gerichtet sind.

8. Einheit gemäß einem der Ansprüche 7, wobei die Permanentmagnete unterhalb des Meniskus einer in einem Reaktionsgefäß, das von dem Thermoblock gehalten wird, enthaltenen Flüssigkeit angeordnet sind.

9. Einheit gemäß einem der Ansprüche 1 bis 8, wobei die Nachweiseinheit eine optische Nachweiseinheit ist.

10. Verfahren zum Bestimmen der Menge eines Analyten, der an magnetische Partikel gebunden ist, die in einem Probenfluid in einem Reaktionsgefäß vorhanden sind, wobei das Verfahren die Schritte umfasst
a) Bereitstellen einer biologischen Probe, die einen Analyten umfasst, in wenigstens einem Reaktionsgefäß;
b) Binden des Analyten an magnetische Partikel in dem wenigstens einen Reaktionsgefäß;
c) Bearbeiten des an magnetische Partikel in dem wenigstens einen Reaktionsgefäß gebundenen Analyten, um ein Nachweissignal zu erhalten, wobei das Nachweissignal ein Signal ist, das durch das Vorhandensein der magnetischen Partikel gestört wird, wobei das wenigstens eine Reaktionsgefäß in einer Einheit gemäß Ansprüchen 1 bis 9 angeordnet ist;
d) Erzeugen eines Überstands durch Anwenden eines Magnetfelds auf den Inhalt des wenigstens einen Reaktionsgefäßes und Absondern der magnetischen Partikel;
e) Nachweisen des Nachweissignals, das einer Analytenkonzentration in dem Überstand des Reaktionsgefäßes entspricht, während die magnetischen Partikel ein Pellet an dem Ort der Seitenwände des Reaktionsgefäßes bilden, an den sie abgesondert wurden,
wobei die Schritte c) bis e) in der gleichen analytischen Bearbeitungseinheit durchgeführt werden und das Reaktionsgefäß in dem Thermoblock der Einheit gemäß Ansprüchen 1 bis 9 angeordnet ist.

11. Verfahren gemäß Anspruch 10, wobei der Analyt eine Nukleinsäure oder ein Polypeptid ist.

12. Verfahren gemäß Ansprüchen 10 und/oder 11, wobei das Bearbeiten der Nukleinsäure Vervielfältigen umfasst.

13. Verfahren gemäß einem der Ansprüche 10 bis 12, wobei das Nachweissignal ein optisches Nachweissignal ist, vorzugsweise Fluoreszenz.

14. Verfahren gemäß einem der Ansprüche 10 bis 13, wobei die Bearbeitung die Elution des Analyten von den magnetischen Kügelchen in das Fluid in dem Reaktionsgefäß umfasst.

15. Verfahren gemäß Anspruch 11, wobei das Bearbeiten des Polypeptids in Schritt c) die Schritte umfasst
c1) Inkontaktbringen des an magnetische Partikel gebundenen Analyten mit einem Nachweismolekül, vorzugsweise einem markierten Antikörper, der den Analyten binden kann, und
c2) Eluieren des an den Analyten gebundenen Nachweismoleküls, um ein Nachweissignal in einem Fluid in dem Reaktionsgefäß zu erhalten.

## Revendications

1. Dispositif de détection et de traitement analytique comprenant
- un bloc thermique pour que vienne s'y loger au moins un récipient de réaction, ledit bloc thermique comprenant au moins une cavité dans laquelle peut venir se loger ledit récipient de réaction ;
- un assemblage magnétique intégré dans ledit bloc thermique, ledit assemblage magnétique comprenant une ou plusieurs unités pour appliquer un champ magnétique sur ledit au moins un récipient de réaction, comprenant des particules magnétiques capables de se lier à un analyte contenu dans un fluide, et dans lequel ledit champ magnétique généré par au moins une unité magnétique donne lieu à une séquestration des particules magnétiques contre la paroi latérale interne dudit au moins un récipient de réaction ; et
- une unité de détection pour détecter un signal correspondant à une concentration d'analyte dans le produit surnageant dudit au moins un récipient de réaction, ladite unité de détection étant mise en alignement avec ledit récipient logé dans ledit bloc thermique en présence des particules magnétiques séquestrées.

2. Dispositif selon la revendication 1, dans lequel la matière dudit bloc thermique comprend de l'aluminium ou de l'argent.

3. Dispositif selon les revendications 1 à 2, dans lequel ledit champ magnétique peut être augmenté ou diminué.

4. Dispositif selon l'une quelconque des revendications 1 à 3, dans lequel lesdites une ou plusieurs unités pour appliquer un champ magnétique sur au moins un récipient de réaction comprenant des particules magnétiques dans le fluide représentent un ou plusieurs aimants permanents qui sont intégrés dans ledit bloc thermique.

5. Dispositif selon la revendication 4, dans lequel lesdits uns ou plusieurs aimants permanents manifestent une stabilité thermique à des températures s'élevant jusqu'à 140 °C, de préférence jusqu'à 180 °C, de manière plus préférée jusqu'à 250 °C.

6. Dispositif selon la revendication 4, dans lequel lesdits uns ou plusieurs aimants permanents comprennent un alliage SmCo.

7. Dispositif selon l'une quelconque des revendications 4 à 6, dans lequel lesdites cavités pour que viennent s'y loger des récipients de réaction dudit bloc thermique sont arrangées entre au moins deux desdits aimants permanents de telle sorte que les pôles magnétiques égaux desdits aimants permanents sont tournés vers les mêmes cavités.

8. Dispositif selon la revendication 7, dans lequel lesdits aimants permanents sont disposés en dessous du ménisque d'un liquide compris dans le récipient de réaction qui est maintenu par ledit bloc thermique.

9. Dispositif selon l'une quelconque des revendications 1 à 8, dans lequel ladite unité de détection est une unité de détection optique.

10. Procédé pour déterminer la quantité d'un analyte lié à des particules magnétiques présentes dans un fluide échantillon dans un récipient de réaction, ledit procédé comprenant les étapes dans lesquelles :
a) on procure un échantillon biologique comprenant un analyte dans au moins un récipient de réaction ;
b) on lie ledit analyte à des particules magnétiques dans ledit au moins un récipient de réaction ;
c) on traite ledit analyte lié à des particules magnétiques dans ledit au moins un récipient de réaction pour obtenir un signal de détection, ledit signal de détection étant un signal qui est perturbé par la présence desdites particules magnétiques, ledit au moins un récipient de réaction étant logé dans un dispositif selon les revendications 1 à 9 ;
d) générer un produit surnageant en appliquant un champ magnétique sur le contenu dudit au moins un récipient de réaction et séquestrer lesdites particules magnétiques ;
e) détecter ledit signal de détection correspondant à une concentration d'analyte dans le produit surnageant dudit récipient de réaction tandis que les particules magnétiques forment une pastille à l'endroit des parois latérales du récipient de réaction auquel elles ont été séquestrées ;
dans lequel les étapes c) à e) sont mises en oeuvre dans la même unité de traitement analytique et le récipient de réaction est disposé dans le bloc thermique du dispositif selon les revendications 1 à 9.

11. Procédé selon la revendication 10, dans lequel ledit analyte est un acide nucléique ou un polypeptide.

12. Procédé selon les revendications 10 et/ou 11, dans lequel le traitement dudit acide nucléique comprend une amplification.

13. Procédé selon l'une quelconque des revendications 10 à 12, dans lequel ledit signal de détection est un signal de détection optique, de préférence une fluorescence.

14. Procédé selon l'une quelconque des revendications 10 à 13, dans lequel ledit traitement comprend une élution dudit analyte à partir des billes magnétiques jusque dans le fluide dans ledit récipient de réaction.

15. Procédé selon la revendication 11, dans lequel ledit traitement dudit polypeptide à l'étape c) comprend les étapes dans lesquelles :
c1) on met ledit analyte lié à des particules magnétiques en contact avec une molécule de détection, de préférence un anticorps marqué qui est à même de se lier audit analyte ; et
c2) on élue ladite molécule de détection liée audit analyte pour obtenir un signal de détection dans un fluide dans lesdits récipients de réaction.
